# EUROPEAN PATENT SPECIFICATION

(11) **EP 3 453 320 B1**
(45) Date of publication and mention of the grant of the patent: **13.12.2023**
(21) Application number: 17789378.1
(22) Date of filing: 19.04.2017
(51) Int. Cl.: A61B 5/02, A61B 5/021, A61B 5/00

(54) **PULSE WAVE DETECTION DEVICE AND BIOLOGICAL INFORMATION MEASUREMENT DEVICE**
PULSWELLENDETEKTIONSVORRICHTUNG UND VORRICHTUNG ZUR MESSUNG BIOLOGISCHER INFORMATIONEN
DISPOSITIF DE DÉTECTION D'ONDES PULSATILES ET DISPOSITIF DE MESURE D'INFORMATIONS BIOLOGIQUES

(30) Priority: 28.04.2016 JP 2016091665
(43) Date of publication of application: 13.03.2019
(73) Proprietor: Omron Healthcare Co., Ltd., Muko-shi, Kyoto 617-0002 (JP)
(72) Inventor: KATO Yuki, Muko-shi Kyoto 617-0002 (JP); MANO Jumpei, Muko-shi Kyoto 617-0002 (JP); FUJITA Reiji, Muko-shi Kyoto 617-0002 (JP); YAMASHITA Shingo, Muko-shi Kyoto 617-0002 (JP); OGURA Toshihiko, Muko-shi Kyoto 617-0002 (JP)
(74) Representative: Isarpatent
(86) International application number: PCT/JP2017/015752
(87) International publication number: WO 2017/188093

(56) References cited:
- WO-A1-2015/115236
- JP-A- H0 467 839
- JP-A- H01 288 228
- JP-A- S57 168 133
- JP-A- 2002 330 932
- JP-A- 2016 014 581
- JP-U- H01 117 303
- US-A- 5 179 956
- US-A1- 2015 011 841

## Description

### Technical Field

The present invention relates to a pulse wave detector and a biological information measurement device.

### Background Art

A biological information measurement device is known that, in a state where a pressure sensor is directly made contact with a living body portion through which an artery such as the radial artery in the wrist passes, can measure biological information such as pulse or blood pressure by using information detected by this pressure sensor (see Patent Literature 1).

The pressure sensor mounted on the biological information measurement device described in Patent Literature 1 is equipped with a circuit board, a spacer provided on this circuit board, a sensor chip provided on this spacer, a case for accommodating the circuit board, and a protection plate for protecting the circuit board. This protection plate is provided with an opening, and the sensor chip protrudes from this opening.

Patent Literature 2 discloses a pulse wave detector comprising a pressure sensor which includes a plate member, a sensor chip positioned spaced to the plate member by a spacer which is arranged between the plate member and the sensor chip, and a case carrying the plate member. A protection plate is attached to a front face of the case and includes an opening through which the spacer protrudes. A silicone filling material is provided so as to cover the sensor chip and so as to fill a gap between an edge of the opening of the protection plate.

Patent Literature 3 discloses a pulse wave detector with a sensor chip. The sensor chip comprises a board having an elongated extended shape in an X direction and disposed so as to intersect an artery. On the board, a pressure sensor array comprising a plurality of pressure sensor elements aligned along the X direction is formed. In a region opposite an end of the pressure sensor array on the board, an electrode terminal array is formed for drawing output of the plurality of pressure sensor elements outside the chip. Regions corresponding to both sides of the pressure sensor array on the board are flat surfaces on which no electrode terminals exist.

Similar pulse wave detectors are also disclosed in U.S. Patent US 5179956 A, and in the published PCT application WO 2015/115236.

### Citation List

### Patent Literature

Patent Literature 1: JP-A-4-67839
Patent Literature 2: US 5 179 956 A
Patent Literature 3: WO 2015/115236 A1

### Summary of Invention

### Technical Problem

In the sensor section described in Patent Literature 1, the sensor chip protrudes from the opening of the protection plate. Hence, the surface of the sensor chip in the pressure sensor dominantly makes contact with the wrist, whereby the force applied to the surface of the sensor chip becomes large. Consequently, in the case that it is assumed that the biological information measurement device is attached for a long time such as a whole day, improving the durability of the sensor chip becomes a problem to be solved.

The present invention has been made in consideration of the above-mentioned circumstances, and an object of the present invention is to provide a pressure pulse wave sensor capable of ensuring sufficient durability in the case of wearing the sensor for a long time and capable of suppressing manufacturing cost, a pulse wave detector, and a biological information measurement device.

### Means for solving Problem

The present invention provides a pulse wave detector in accordance with claim 1 and a biological information measurement device in accordance with claim 6.

A pressure pulse wave sensor according to examples of the present disclosure is equipped with sensor chips having pressure detection elements; a substrate to which the sensor chips are fixed; and a protection member for protecting the substrate and the sensor chips, wherein, in the vertical direction perpendicular to the detection faces of the sensor chips on which the pressure detection elements are formed, the protection member has opening sections disposed at positions opposed to the detection faces on the side opposite to the side of the fixture portion of the sensor chips and the substrate rather than the side of the pressure detection elements, and the space between the opening sections and the detection faces is filled with a filling material.

The pulse wave detector according to the present invention is equipped with the pressure pulse wave sensor, the sensor chip of the pressure pulse wave sensor has an element row including the plurality of pressure detection elements arranged in one direction, and the outer peripheral face of the protection member is a face continuing to both the edges in the one direction of the top face in which the opening sections are formed and has inclined faces inclined with respect to the opening faces of the opening sections; the pulse wave detector is further equipped with an air bag and a rotation mechanism fixed to the air bag for rotating the pressure pulse wave sensor around the respective directions orthogonal to the one direction and the vertical direction, and the inclination angle of the inclined faces is set to a value greater than the maximum value of the rotation angle of the pressure pulse wave sensor capable of being rotated by the rotation mechanism.

The biological information measurement device according to the present invention is equipped with the pulse wave detector and a biological information calculation section for calculating biological information on the basis of the pressure pulse wave detected by the pressure pulse wave sensor.

The biological information measurement device according to the present invention is equipped with the pressure pulse wave sensor and the biological information calculation section for calculating biological information on the basis of the pressure pulse wave detected by the pressure pulse wave sensor.

### Advantageous Effects of Invention

The present invention can provide a pressure pulse wave sensor capable of ensuring sufficient durability in the case of wearing the sensor for a long time and capable of suppressing manufacturing cost and a biological information measurement device equipped with this sensor.

### Brief Description of Drawings

[Fig. 1] Fig. 1 is a schematic view showing the outline configuration of a biological information measurement device 100 according to an embodiment of the present invention.
[Fig. 2] Fig. 2 is an exploded perspective view showing the external configuration of the pressure pulse wave sensor 10 shown in Fig. 1.
[Fig. 3] Fig. 3 is a perspective view showing the outline configuration of the pressure pulse wave sensor 10 shown in Fig. 1.
[Fig. 4] Fig. 4 is a schematic view showing the external configuration of the sensor section 20 shown in Fig. 2.
[Fig. 5] Fig. 5 is a sectional schematic view taken on line V-V in Fig. 3.
[Fig. 6] Fig. 6 is a plan view showing the pressure pulse wave sensor 10 shown in Fig. 3, as viewed in the Z-direction.
[Fig. 7] Fig. 7 is a perspective view showing the external configuration of a protection cover 401, a modification of the protection cover 40 of the pressure pulse wave sensor 10 shown in Fig. 2.
[Fig. 8] Fig. 8 is a sectional schematic view showing a pressure pulse wave sensor 12, a modification of the pressure pulse wave sensor 10 shown in Fig. 2.
[Fig. 9] Fig. 9 is a perspective view showing the external configuration of a sensor section 201, a modification of the sensor section 20 of the pressure pulse wave sensor 10 shown in Fig. 2.
[Fig. 10] Fig. 10 is a sectional schematic view showing a modification of the pressure pulse wave sensor 10 shown in Fig. 2.

### Description of Embodiments

An embodiment of the present invention will be described below referring to the drawings.

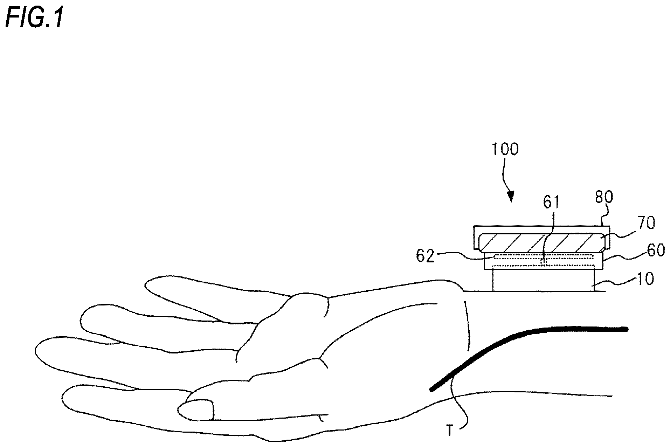

Fig. 1 is a schematic view showing the outline configuration of a biological information measurement device 100 according to an embodiment of the present invention.

The biological information measurement device 100 is equipped with a housing 80, an air bag 70 supported by the housing 80, a rotation driving section 60 fixed to the air bag 70, and a pressure pulse wave sensor 10 rotatably supported by the rotation driving section 60. The biological information measurement device 100 is attached by a band, not shown, to a wrist of a person to be measured.

The air bag 70 constitutes a pressing member for pressing the rotation driving section 60 and the pressure pulse wave sensor 10 against the body surface of the wrist. The internal pressure of the air bag 70 is adjusted by a pump, not shown, built in the housing 80. The pump is controlled by a control section that is built in the housing 80.

The rotation driving section 60 has a rotation mechanism 61 for rotating the pressure pulse wave sensor 10 around the axes respectively extending in two directions orthogonal to each other along the detection face, described later, of the pressure pulse wave sensor 10 and an actuator 62 for driving the rotation mechanism 61. The actuator 62 is controlled by the control section built in the housing 80.

The pressure pulse wave sensor 10 is a sensor for detecting a pressure pulse wave serving as a pressure oscillating wave and transmitted from the radial artery T passing through the inside of the wrist to the body surface in the state in which the pressure pulse wave sensor 10 is pressed by the air bag 70 against the body surface with a predetermined pressing force. The housing 80 incorporates a biological information calculation section, not shown, for calculating biological information, such as pulse rate, heart rate or blood pressure value, on the basis of the pressure pulse wave detected by the pressure pulse wave sensor 10.

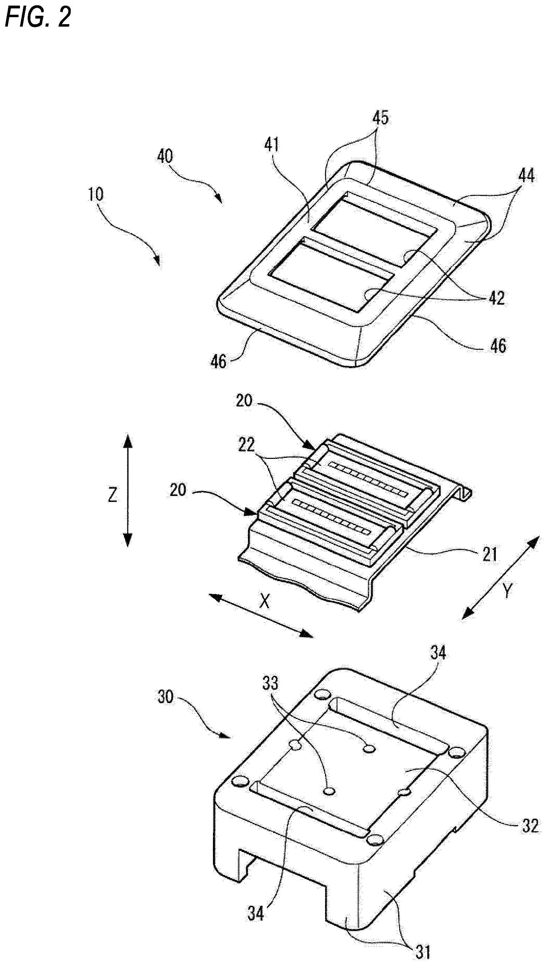

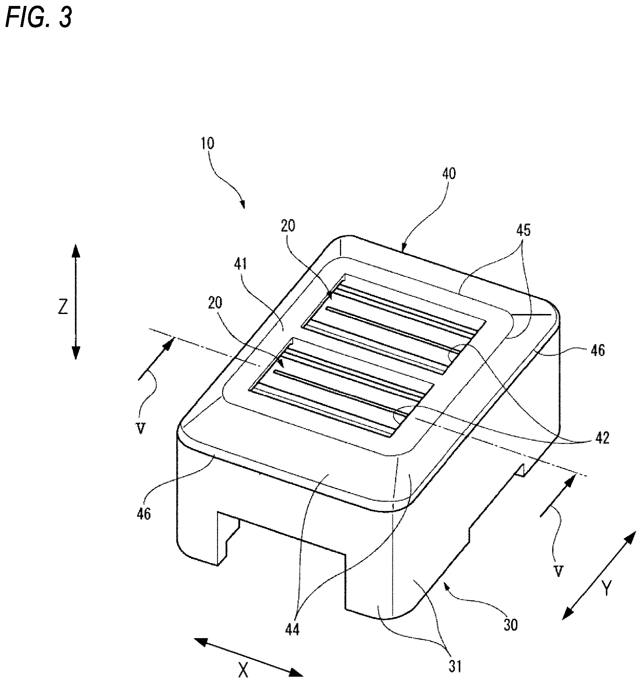

Fig. 2 is an exploded perspective view showing the external configuration of the pressure pulse wave sensor 10 shown in Fig. 1. Fig. 3 is a perspective view showing the outline configuration of the pressure pulse wave sensor 10 shown in Fig. 1.

The pressure pulse wave sensor 10 is equipped with two sensor sections 20 having a longitudinal shape along the X-direction, one of two directions, a flexible substrate 21 to which the two sensor sections 20 are fixed in the Y-d orthogonal to the X-direction, a base 30 to which the flexible substrate 21 is fixed, and a protection cover 40 that is fixed to the base 30 to which the flexible substrate 21 is fixed so as to serve as a protection member for protecting the two sensor sections 20.

The base 30 has a rectangular planar shape as viewed in the Z-direction orthogonal to the X-direction and Y-direction and has a mounting face 32 on which the flexible substrate 21 is mounted. Two base through-holes 33 passing through the mounting face 32 in the Z-direction are provided in the mounting face 32; the number thereof corresponds to the number of the sensor sections 20. Furthermore, openings 34 having a size allowing the flexible substrate 21 to be inserted are provided at both the end positions in the Y-direction of the mounting face 32. Both the ends of the flexible substrate 21 are pulled out to the rear side of the base 30 through these openings 34.

The planar shape of the protection cover 40 as viewed in the Z-direction is a rectangular shape similar to the external shape of the base 30 and has a size similar to the external shape thereof, and in the state in which the flexible substrate 21 is fixed to the base 30, the protection cover 40 is fixed to the peripheral face of the mounting face 32 of the base 30.

In the state of being fixed to the base 30, the protection cover 40 has two opening sections 42 formed respectively at positions opposed to the two sensor sections 20. The outer peripheral face (the face exposed to the outside in the state shown in Fig. 3) of the protection cover 40 has a flat top face 41, inclined faces 44 continuing to the edges 45 of the top face 41, and vertical faces 46 continuing to the edges of the inclined faces 44 and perpendicular to the top face 41. The detailed shape of the protection cover 40 will be described later.

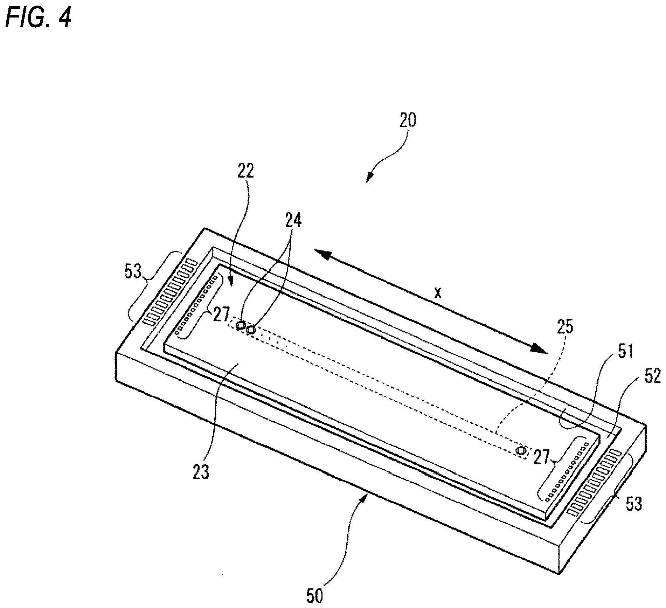

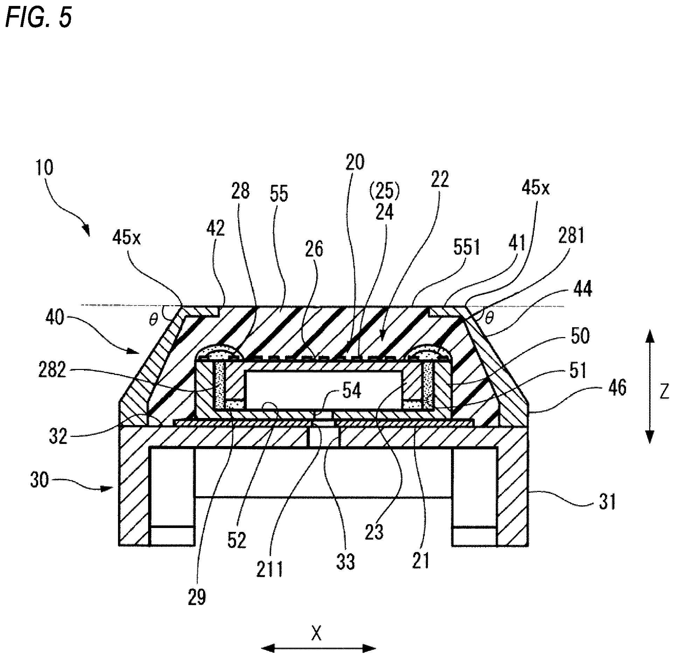

Fig. 4 is a schematic view showing the external configuration of the sensor section 20 shown in Fig. 2. Fig. 5 is a sectional schematic view taken on line V-V in Fig. 3.

The sensor section 20 is equipped with a sensor chip 22 and a container-like substrate 50 having a concave section 51, wherein the sensor chip 22 is fixed to the bottom face 52 of the concave section 51.

The sensor chip 22 has a semiconductor substrate 23 made of a single crystal of silicon or a single crystal of a compound semiconductor, such as gallium arsenide. The semiconductor substrate 23 is formed into a rectangular shape so that the longitudinal direction thereof is oriented in the X-direction. An element row 25 including a plurality of pressure detection elements 24 arranged along the X-direction is formed on the surface of the semiconductor substrate 23. The pressure detection element 24 is an element for detecting the strain applied to the element as a pressure signal, and for example, a strain gauge resistance type element, a semiconductor piezo resistance type element or an electrostatic capacitance type element is used. The surface of the semiconductor substrate 23 is a flat face and this flat face constitutes a detection face 26 for detecting pressure. The direction perpendicular to the detection face 26 is the Z-direction.

A chip-side terminal section 27 including electrode pads electrically connected to the respective pressure detection elements 24 is formed at each of both the end sections in the X-direction of the surface of the semiconductor substrate 23 (see Fig. 4).

The substrate 50 is formed of a hard substrate, such as a ceramic substrate or a glass substrate, having rigidity sufficiently higher than that of the semiconductor substrate 23. The substrate 50 is formed into a rectangular shape so that the longitudinal direction thereof is oriented in the X-direction.

As shown in Fig. 5, in the face of the semiconductor substrate 23 opposite to the detection face 26, a concave section recessed in the Z-direction perpendicular to the detection face 26 is formed. By virtue of this concave section, the semiconductor substrate 23 is configured so as to have a thin section (diaphragm), the thickness of which is smaller than that of the other portion in the Z-direction.

The portion of the face of the semiconductor substrate 23 opposite to the detection face 26 excluding the concave section is fixed to the bottom face 52 of the concave section 51 of the substrate 50 with an adhesive 29. As the adhesive 29, an ultraviolet curing resin is used for example.

The semiconductor substrate 23 is fixed to the bottom face 52 of the concave section 51 so that the concave section of the semiconductor substrate 23 communicates with the atmosphere only through the through holes 54 formed in the bottom face of the concave section 51 of the substrate 50.

As shown in Fig. 5, in the flexible substrate 21, through holes 211 are formed at the positions opposed to the through holes 54 of the substrate 50. These through holes 211 communicate with the base through holes 33 of the base 30. Hence, the inside of the concave section of the semiconductor substrate 23 is kept at the atmospheric pressure by the base through holes 33, the through holes 211, and the through holes 54.

As shown in Fig. 4, at each of both the end sections in the X-direction of the substrate 50, a substrate-side terminal section 53 including electrode pads for electrical connection to the respective electrode pads of the chip-side terminal section 27 is provided on the face of the substrate 50 in which the concave section 51 is formed.

As shown in Fig. 5, the electrode pads of the chip-side terminal section 27 and the electrode pads of the substrate-side terminal section 53 are connected via conductive members 28 made of gold, aluminum or the like. The conductive members 28 are covered therearound and protected with protection members 281 made of an insulation material, such as resin. The space between the side face of the concave section 51 of the substrate 50 and both the semiconductor substrate 23 and the adhesive 29 is filled with a material 282 that is less changed in volume than the protection member 281 depending on temperature and humidity.

Next, the details of the protection cover 40 will be described.

In the Z-direction perpendicular to the detection face 26 of the sensor chip 22, the top face 41 of the protection cover 40 is disposed on the side opposite to the side of the fixture portion (the place where the adhesive 29 is located) of the sensor chips 22 and the substrate 50 rather than the side of the pressure detection elements 24, and the top face 41 is parallel with the detection face 26. The state in which the two faces are parallel with each other is the state in which the angle formed by the two faces is within the range of a tolerance with 0° taken as its center. Similarly, the state in which the two faces are perpendicular with each other is the state in which the angle formed by the two faces is within the range of a tolerance with 90° taken as its center.

The opening sections 42 each passing through the position opposed to the detection face 26 of each sensor chip 22 to the detection face 26 are formed in the top face 41 of the protection cover 40. The element row 25 entirely overlaps with the opening section 42 in a plan view in the Z-direction.

In the space surrounded by the inner peripheral face of the protection cover 40, the base 30, the sensor sections 20 and the flexible substrate 21 is filled with a filling material 55 as shown in Fig. 5.

The filling material 55 is also used to fill the opening sections 42 of the protection cover 40, and the surface 551 of the filling material 55 exposed to the outside is formed so as to be flush with the top face 41 of the protection cover 40. The surface 551 constitutes the opening faces of the opening sections 42. The flat face formed by the surface 551 of the filling material 55 and the top face 41 of the protection cover 40 is parallel with the detection faces 26 and forms a contact face making contact with the body surface of the person to be measured.

It is preferable that a material having hardness lower than that of the protection cover 40 should be used for the filling material 55. For example, ceramic is preferably used as the material of the protection cover 40, and an epoxy resin, a silicon resin or the like having hardness lower than that of ceramic is used as the filling material 55.

As shown in Fig. 5, in the cross section along the straight line that overlaps with the top face 41 and extending in the X-direction, the inclined faces 44 of the protection cover 40 continue to both the edges 45x in the X-direction of the top face 41 and are inclined faces inclined at an inclination angle θ with respect to the face 551 so as to approach the sensor chips 22 in the Z-direction.

Furthermore, as shown in Fig. 5, the vertical faces 46 of the protection cover 40 are faces continuing to the inclined faces 44 and perpendicular to the surface 551. The vertical faces 46 are located on the side of the sensor chips 22 rather than the side of the surface 551 in the Z-direction and located outside the edges 45 of the top face 41 in a plan view as viewed in the Z-direction.

The shape of the protection cover 40 in the cross section along the straight line extending in the Y-direction of the pressure pulse wave sensor 10 shown in Fig. 3 and passing through the top face 41 is similar to the shape of the protection cover 40 shown in Fig. 5. In other words, the outer peripheral face of the protection cover 40 is a face continuing to both the edges in the Y-direction of the top face 41, and has the inclined faces 44 inclined with respect to the top face 41 so as to approach the sensor chips 22 and the vertical faces 46 continuing to the inclined faces 44.

The respective side faces 31 of both the end sections of the base 30 in the X-direction and the Y-direction are formed so as to continue to the vertical faces 46 of the protection cover 40 without level difference in the state in which the protection cover 40 is fixed, and the vertical faces 46 and the side faces 31 are formed into the same face being parallel with the Z-direction.

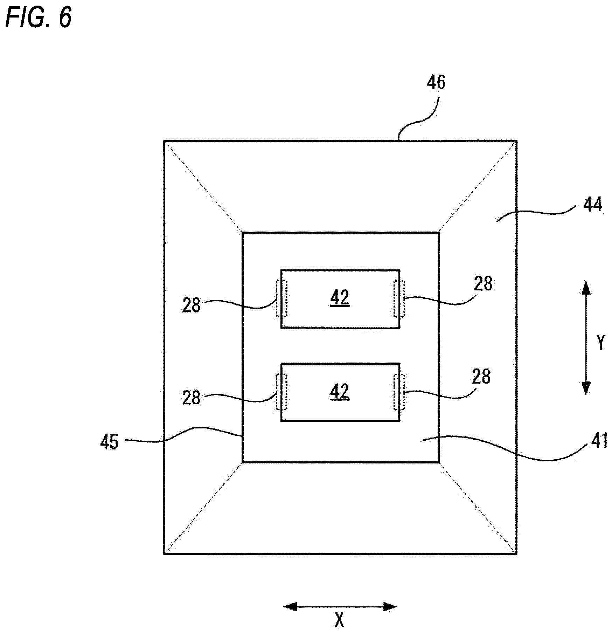

Fig. 6 is a plan view showing the pressure pulse wave sensor 10 shown in Fig. 3, as viewed in the Z-direction. In Fig. 6, only the positions of the conductive members 28 of the sensor sections 20 are shown, and the other components are omitted.

As shown in Fig. 6, the conductive members 28 of the sensor sections 20 are disposed at positions that do not overlap with the edges 45 of the top face 41 in a plan view as viewed in the Z-direction.

The edges 45 of the top face 41 are portions to which the largest stress is applied in the state in which the top face 41 is pressed against the body surface. On the other hand, since the conductive members 28 are formed of, for example, wire bonding, if a large force is applied thereto, wire breakage may occur.

As shown in Fig. 6, since the conductive members 28 are located at positions that do not overlap with the edges 45 of the top face 41, the conductive members 28 are not located at positions below the portions to which the large stress is applied. Hence, even in the case that the pressure pulse wave sensor 10 is pressed against the body surface, the force to be applied to the conductive members 28 can be decreased, whereby wire breakage can be prevented.

Although the conductive members 28 are located inside the edges 45, an effect similar to that described above can be obtained even in a configuration in which the conductive members 28 are located outside the edges 45.

The rotation mechanism 61 of the biological information measurement device 100 shown in Fig. 1 is a mechanism for rotating the pressure pulse wave sensor 10 around the respective two axes extending in the X-direction and the Y-direction.

The biological information measurement device 100 configured as described above is attached to a wrist so that the X-direction intersects the radial artery T (see Fig. 1) of the wrist from which a pressure pulse wave is detected.

After the biological information measurement device 100 is attached to the wrist and a measurement start instruction is issued, the control section increases the internal pressure of the air bag 70, thereby pressing the pressure pulse wave sensor 10 against the surface of the wrist.

The control section determines the rotation angles around the respective two axes extending in the X-direction and the Y-direction of the pressure pulse wave sensor 10 so that the detection accuracy of a pressure pulse wave becomes maximum.

The control section rotates the pressure pulse wave sensor 10 by the rotation angles determined as described above, detects a pressure pulse wave using the pressure detection elements 24 of the pressure pulse wave sensor 10 in the state in which the pressure pulse wave sensor 10 is pressed against the body surface with a predetermined pressing force, calculates biological information on the basis of the detected pressure pulse wave, and stores the biological information.

As described above, with the pressure pulse wave sensor 10, the opening sections 42 of the protection cover 40 are provided above the detection faces 26 of the sensor chips 22, and the opening sections 42 are filled with the filling material 55, whereby the contact face making contact with the body surface is formed by the top face 41 of the protection cover 40 and the surface 551 of the filling material 55.

Since the contact face to be pressed against the body surface includes the protection cover 40 and the filling material 55 as described above, the durability of the sensor chips 22 of the pressure pulse wave sensor 10 can be improved. Hence, the biological information measurement device 100 can be used for a long time while being attached to the wrist. The durability can be further improved by using a material having hardness higher than that of the filling material 55 as the material of the protection cover 40.

Moreover, with the pressure pulse wave sensor 10, the surface 551 of the filling material 55 can be made flat easily by using the flatness of the top face 41. Hence, the manufacturing cost thereof can be reduced. In the case that a material having hardness higher than that of the filling material 55 is used as the material of the protection cover 40, the surface 551 of the filling material 55 can be made flat more easily.

What's more, the pressure pulse wave sensor 10 has a configuration in which the protection cover 40 has the inclined faces 44 continuing to both the edges 45x in the X-direction of the top face 41. With this configuration, in the case that the pressure pulse wave sensor 10 is rotated around the axis extending in the Y-direction (around the wrist) by the rotation driving section 60, the area of the protection cover 40 making contact with the body surface can be reduced.

The biological information measurement device 100 is attached to the wrist so that the top face 41 of the protection cover 40 is located above the radial artery T; however, hard tissues, such as a radius and a tendon, exist around the radial artery T.

In the case that the pressure pulse wave sensor 10 is rotated around the wrist, since the inclined faces 44 of the protection cover 40 can release the pressure from these hard tissues, the feeling of wearing the biological information measurement device 100 can be improved. Furthermore, since the rotation operation of the pressure pulse wave sensor 10 is hardly hindered by the hard tissues, the desired rotation angle can be maintained by small driving power and pressing force.

The inclined faces 44 of the protection cover 40 may be curved faces instead of the flat faces. In other words, the protection cover 40 may be configured so as to have curved faces connecting the top face 41 to the vertical faces 46. The curved faces constitute faces continuing to both the edges in the X-direction of the top face 41 and intersecting the surface 551 and the respective faces perpendicular to the surfaces 551. Even in the case that the inclined faces 44 are formed of the curved faces as described above, in the case that the pressure pulse wave sensor 10 is rotated around the wrist, since the curved faces of the protection cover 40 can release the pressure applied from the hard tissues, the feeling of wearing the biological information measurement device 100 can be improved.

The inclination angle θ of the inclined face 44 shown in Fig. 5 is preferably set to a value greater than the maximum value of the rotation angle (the rotation angle at the time when the state in which the detection face 26 is perpendicular to the direction of pressing the pressure pulse wave sensor 10 by the air bag 70 is used as a reference) by which the pressure pulse wave sensor 10 can be rotated around the axis extending in the Y-direction by the rotation mechanism 61.

With the setting of the value as described above, even in the case that the pressure pulse wave sensor 10 is rotated around the wrist to the maximum, the inclined faces 44 are prevented from making contact with the body surface, and the rotation operation can be performed smoothly.

In addition, the pressure pulse wave sensor 10 is configured so that the protection cover 40 has the vertical faces 46. In order to acquire calibration data required for converting the strain signals detected by the pressure detection elements 24 into a pressure value, the sensor chip 22 requires to perform a work of acquiring the signals from the pressure detection elements 24 by applying pressure to the contact face in the state in which the contact face constituting the top face 41 and the surface 551 is accommodated in a sealed container in a manufacturing process.

In the protection cover 40, since the vertical faces 46 are provided on the side of the base 30 rather than the side of the surface 551, in the case that a cap-shaped device is installed around the vertical faces 46, the interior of the device can be made hermetically sealed easily. Hence, the work for generating the calibration data can be made easy and the manufacturing cost can be reduced.

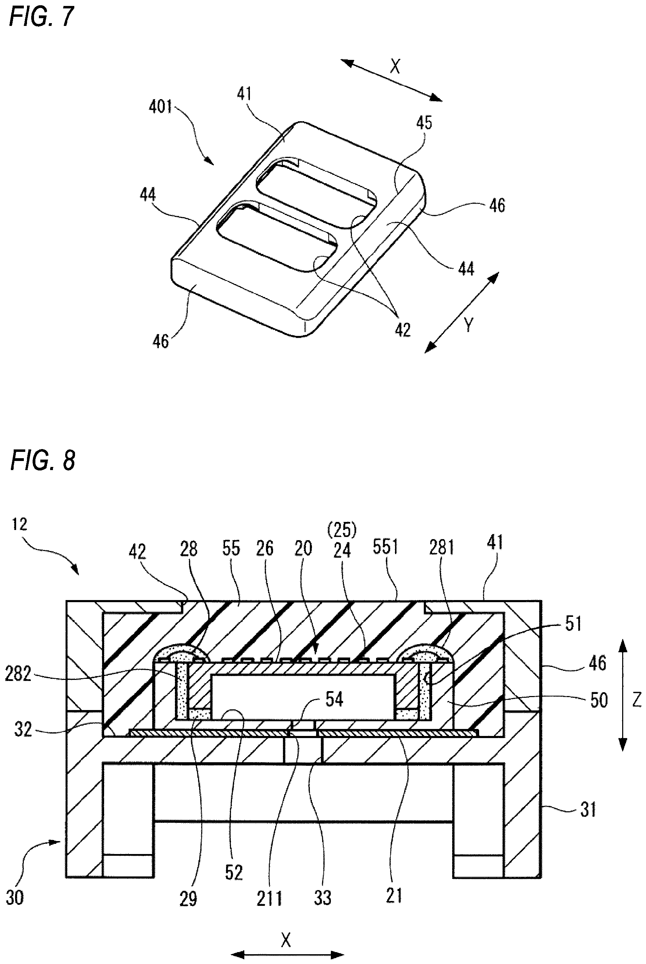

Fig. 7 is a perspective view showing the external configuration of a protection cover 401, a modification of the protection cover 40 of the pressure pulse wave sensor 10 shown in Fig. 2.

The protection cover 401 is configured such that both the end faces in the Y-direction of the protection cover 40 are changed to the vertical faces 46 perpendicular to the surface 551. The vertical faces 46 are faces continuing to the edges in the Y-direction of the top face 41.

With the configuration of the protection cover 40, since the edges in the Y-direction of the top face 41 are located away from the sensor chips 22, the stresses applied to the sensor chips 22 can be reduced, whereby the detection accuracy of the pressure pulse wave can be improved, and the durability of the pressure pulse wave sensor 10 can be improved.

The outer peripheral face of the protection cover 40 of the pressure pulse wave sensor 10 may include only the top face 41 and the vertical faces 46 continuing to the edges 45 of the top face 41 and perpendicular to the surface 551. In other words, the inclined faces 44 on the outer peripheral face of the protection cover 40 are not essential.

Fig. 8 is a sectional schematic view showing a pressure pulse wave sensor 12, a modification of the pressure pulse wave sensor 10 shown in Fig. 2.

The configuration of the pressure pulse wave sensor 12 shown in Fig. 8 is different from the configuration shown in Fig. 5 in that the outer peripheral face of the protection cover 40 includes the top face 41 and the vertical faces 46 continuing to the edges of the top face 41 and that the outer peripheral face of the protection cover 40 does not have the inclined faces 44. In the other sections of the configuration, the pressure pulse wave sensor 12 is similar to the pressure pulse wave sensor 10 and a working effect similar thereto can be obtained. In the configuration shown in Fig. 8, the edges of the top face 41 overlap with the vertical faces 46 in a plan view as viewed in the Z-direction.

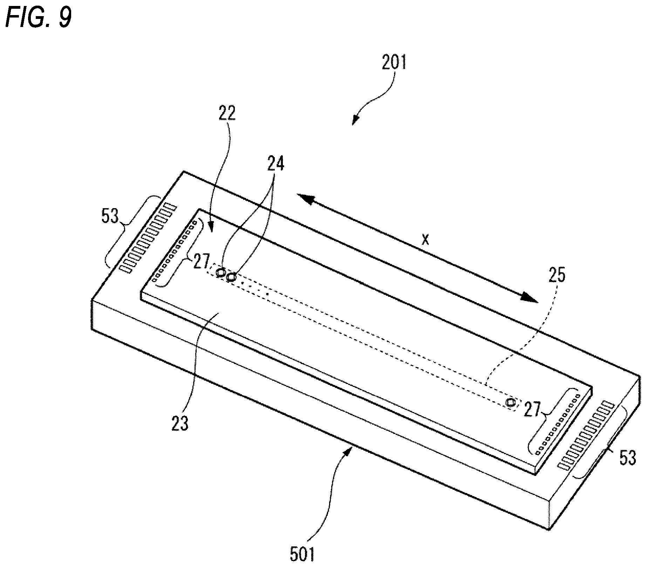

The configuration of the sensor section 20 to be mounted on the pressure pulse wave sensor 10 and the pressure pulse wave sensor 12 is not limited to those shown in Figs. 1 and 8. For example, the sensor section 20 may be changed to the sensor section 201 shown in Fig. 9.

The configuration of the sensor section 201 is the same as that of the sensor section 20 except that the substrate 50 is modified to a substrate 501 having a flat plate shape. Even in the case that the sensor section 201 configured as described above is used, the above-mentioned effects can be obtained.

The presently disclosed embodiment should be considered in all respects to be illustrative and not restrictive. The scope of the invention is indicated by the appended claims rather than the above description, and all changes which come within the meaning thereof are intended to be included therein.

For example, although the pressure pulse wave sensor 10 is assumed to have two sensor sections 20, the pressure pulse wave sensor 10 may have only one sensor section 20. In this case, the rotation mechanism 61 is changed to a mechanism for rotating the pressure pulse wave sensor 10 only around the axis extending in the Y-direction.

In the case of the configuration in which the pressure pulse wave sensor 10 has only one sensor section 20, only one pressure detection element 24 may be formed on the surface of the sensor chip 22 of the sensor section 20. In this case, the rotation driving section 60 can be omitted.

Furthermore, the pressure pulse wave sensor 10 may be configured so as to have three or more sensor sections 20 arranged in the Y-direction.

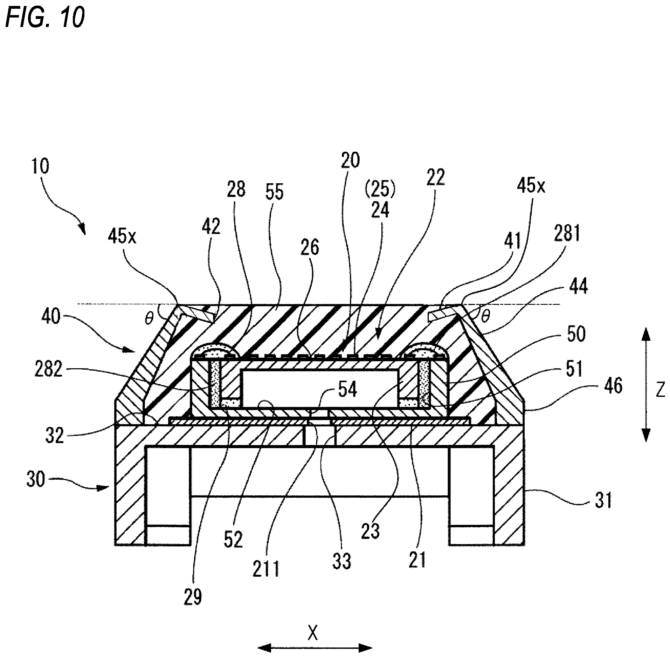

Moreover, although the top face 41 of the protection cover 40 is formed into a flat face that is parallel with the detection faces 26, the top face 41 of the protection cover 40 may be inclined with respect to the detection faces 26 as shown in Fig. 10. Also in the modification shown in Fig. 10, the opening sections 42 formed in the top face 41 are filled with the filling material 55, and the opening faces of the opening section 42 are parallel with the detection faces 26. Even with the configuration described above, the durability can be improved.

Although the biological information measurement device 100 to be attached to a wrist and used has been described above, the present invention can be applied to a type of biological information measurement device that is attached to a living body portion where an artery passes.

As described above, the following matters are disclosed in the Description.

The disclosed pressure pulse wave sensor is equipped with sensor chips having pressure detection elements; a substrate to which the sensor chips are fixed; and a protection member for protecting the substrate and the sensor chips, wherein, in the vertical direction perpendicular to the detection faces of the sensor chips on which the pressure detection elements are formed, the protection member has opening sections disposed at positions opposed to the detection faces on the side opposite to the side of the fixture portion of the sensor chips and the substrate rather than the side of the pressure detection elements, and the space between the opening sections and the detection faces is filled with a filling material.

In the disclosed pressure pulse wave sensor, the protection member is made of a material having hardness higher than that of the filling material.

In the disclosed pressure pulse wave sensor, the protection member is made of ceramic.

In the disclosed pressure pulse wave sensor, the outer peripheral face of the protection member has vertical faces perpendicular to the opening faces of the opening sections, and the vertical faces are located on the side of the sensor chips rather than the side of the opening face in the vertical direction and overlap with the edges of the top face of the protection member in which the opening sections are formed or located outside the edges of the top face in a plan view as viewed in the vertical direction.

In the disclosed pressure pulse wave sensor, the sensor chip has an element row including the plurality of pressure detection elements arranged in one direction, and the outer peripheral face of the protection member is a face continuing to both the edges in the one direction of the top face in which the opening sections are formed and has faces intersecting the opening faces of the opening sections and the respective faces perpendicular to the opening faces.

The disclosed pressure pulse wave sensor is further equipped with substrate-side terminal sections provided on the substrate and used for electrical connection to the terminal sections provided at the ends in the one direction of the sensor chips and conductive members for connecting the terminal sections of the sensor chips and the substrate-side terminal sections, wherein the conductive members are disposed at positions that do not overlap with the edges of the top face in a plan view as viewed in the vertical direction.

In the disclosed pressure pulse wave sensor, the outer peripheral face of the protection member is a face continuing to both the edges in the respective directions orthogonal to the one direction and the vertical direction of the top face and has vertical faces perpendicular to the opening faces of the opening sections.

The disclosed pulse wave detector is equipped with the pressure pulse wave sensor and a rotation mechanism for rotating the pressure pulse wave sensor around the directions respectively orthogonal to the one direction and the vertical direction, wherein the continuing face has inclined faces inclined with respect to the opening faces, the inclination angle of the inclined faces is set to a value greater than the maximum value of the rotation angle of the pressure pulse wave sensor capable of being rotated by the rotation mechanism.

The disclosed biological information measurement device is equipped with the pulse wave detector and a biological information calculation section for calculating biological information on the basis of the pressure pulse wave detected by the pressure pulse wave sensor.

The disclosed biological information measurement device is equipped with the pressure pulse wave sensor and the biological information calculation section for calculating biological information on the basis of the pressure pulse wave detected by the pressure pulse wave sensor.

### Industrial Applicability

The present invention is highly convenient and effective when applied particularly to a wrist-attached sphygmomanometer and the like.

Although the invention has been described with reference to the specific embodiment, the invention is not limited to the embodiment, but it is defined by the appended claims.

The present application is based on Japanese Patent Application (No. 2016-091665) filed on April 28, 2016.

### Reference Signs List

- 100: biological information measurement device
- 10, 12: pressure pulse wave sensor
- 60: rotation driving section
- 61: rotation mechanism
- 62: actuator
- 70: airbag
- 80: housing
- T: radial artery
- 20: sensor section
- 21: flexible substrate
- 211: through hole
- 22: sensor chip
- 23: semiconductor substrate
- 24: pressure detection element
- 25: element row
- 26: detection face
- 27: chip-side terminal section
- 28: conductive member
- 281: protection member
- 282: material
- 29: adhesive
- 30: base
- 31: side face
- 32: mounting face
- 33: base through-hole
- 34: opening
- 40, 401: protection cover
- 41: top face
- 42: opening section
- 44: inclined face
- 45,45x: edge
- 46: vertical face
- 50, 501: substrate
- 51: concave section
- 52: bottom face
- 53: substrate-side terminal section
- 54: through hole
- 55: filling material
- 551: surface (opening face)
- θ: inclination angle
- X, Y, Z: direction

## Claims

1. A pulse wave detector comprising:
a pressure pulse wave sensor (10, 12) including:
sensor chips (22) each having an element row (25) including a plurality of pressure detection elements (24) arranged in one direction;
a substrate (50) to which the sensor chips (22) are respectively fixed;
a protection member (40) for protecting the substrate (50) and the sensor chips (22);
an air bag (70);
a rotation driving section (60) fixed to the air bag (70) and rotatably supporting the pressure pulse wave sensor (10, 12);
and
a filling material (55) being filled in a space between detection faces (26) of the sensor chips (22) on which the pressure detection elements (24) are formed and opening sections (42) provided in the protection member (40), the opening sections (42) being disposed on a side opposite to a side of the sensor chips (22) to which the substrate (50) is fixed and being disposed at positions opposed to the detection faces (26) in a vertical direction perpendicular to the detection faces (26),
wherein the rotation driving section (60) includes a rotation mechanism (61) for rotating the pressure pulse wave sensor (10, 12) around a direction orthogonal to each of the one direction and the vertical direction, wherein
an outer peripheral face of the protection member (40) includes:
a top face (41) in which the opening sections (42) are formed; and
inclined faces (44) that continue to both edges (45x) in the one direction of the top face (41) and are inclined with respect to opening faces of the opening sections (42), inclination angles (θ) of the inclined faces (44) with respect to the opening faces being set to a value greater than a maximum value of a rotation angle of the pressure pulse wave sensor (10, 12) capable of being rotated by the rotation mechanism (61).

2. The pulse wave detector according to claim 1, wherein
the protection member (40) is made of a material having hardness higher than hardness of the filling material (55).

3. The pulse wave detector according to claim 2, wherein
the protection member (40) is made of ceramic.

4. The pulse wave detector according to any one of claims 1 to 3, wherein
the outer peripheral face of the protection member (40) further comprises vertical faces (46) perpendicular to the opening faces of the opening sections (42), and
the vertical faces (46) are located on a side of the sensor chips (22) rather than a side of the opening faces in the vertical direction, and are overlapped with edges of the top face of the protection member or (40) located outside the edges (45,45x) of the top face (41) in a plan view as viewed in the vertical direction.

5. The pulse wave detector according to any one of claims 1 to 4, further comprising:
substrate-side terminal sections (53) for electrically connecting to terminal sections (27) provided at ends in the one direction of the sensor chips (22), the substrate side terminal sections (53) being provided on the substrate (50); and
conductive members (28) for connecting the terminal sections (27) of the sensor chips (22) and the substrate-side terminal sections (53), the conductive members (28) being disposed at positions that do not overlap with the edges (45, 45x) of the top face (41) in a plan view as viewed in the vertical direction.

6. A biological information measurement device (100) comprising:
the pulse wave detector according to any one of claims 1 to 5; and
a biological information calculation section for calculating biological information based on pressure pulse waves detected by the pressure pulse wave sensor (10, 12).

## Patentansprüche

1. Pulswellendetektor, umfassend:
einen Druckimpulswellensensor (10, 12), aufweisend:
Sensorchips (22), die jeweils eine Elementreihe (25) mit mehreren Druckerfassungselementen (24) aufweisen, die in einer Richtung angeordnet sind;
ein Substrat (50), an dem die Sensorchips (22) jeweils befestigt sind;
ein Schutzelement (40) zum Schützen des Substrats (50) und der Sensorchips (22);
einen Airbag (70);
einen Drehantriebsabschnitt (60), der an dem Airbag (70) befestigt ist und den Druckpulswellensensor (10, 12) drehbar trägt; und
ein Füllmaterial (55), das in einen Raum zwischen Erfassungsflächen (26) der Sensorchips (22), auf denen die Druckerfassungselemente (24) gebildet sind, und Öffnungsabschnitten (42), die in dem Schutzelement (40) vorgesehen sind, eingefüllt ist, wobei die Öffnungsabschnitte (42) auf einer Seite angeordnet sind, die einer Seite der Sensorchips (22), an der das Substrat (50) befestigt ist, gegenüberliegt, und an Positionen angeordnet sind, die den Erfassungsflächen (26) in einer vertikalen Richtung senkrecht zu den Erfassungsflächen (26) gegenüberliegen,
wobei der Drehantriebsabschnitt (60) einen Drehmechanismus (61) zum Drehen des Druckpulswellensensors (10, 12) um eine Richtung orthogonal zu sowohl der einen Richtung als auch der vertikalen Richtung aufweist, wobei
eine äußere Umfangsfläche des Schutzelements (40) aufweist:
eine Deckfläche (41), in der die Öffnungsabschnitte (42) gebildet sind; und
geneigte Flächen (44), die sich zu beiden Kanten (45x) in der einen Richtung der Deckfläche (41) fortsetzen und in Bezug auf Öffnungsflächen der Öffnungsabschnitte (42) geneigt sind, wobei Neigungswinkel (θ) der geneigten Flächen (44) in Bezug auf die Öffnungsflächen auf einen Wert eingestellt sind, der größer ist als ein Maximalwert eines Drehwinkels des Druckpulswellensensors (10, 12), der durch den Drehmechanismus (61) gedreht werden kann.

2. Pulswellendetektor nach Anspruch 1, wobei
das Schutzelement (40) aus einem Material hergestellt ist, dessen Härte höher ist als die Härte des Füllmaterials (55).

3. Pulswellendetektor nach Anspruch 2, wobei
das Schutzelement (40) aus Keramik hergestellt ist.

4. Pulswellendetektor nach einem der Ansprüche 1 bis 3, wobei
die äußere Umfangsfläche des Schutzelements (40) ferner vertikale Flächen (46) umfasst, die senkrecht zu den Öffnungsflächen der Öffnungsabschnitte (42) sind, und
die vertikalen Flächen (46) eher auf einer Seite der Sensorchips (22) als auf einer Seite der Öffnungsflächen in der vertikalen Richtung angeordnet sind und mit Kanten der Deckfläche des Schützenelements (40) überlappen oder sich in einer Draufsicht in der vertikalen Richtung gesehen außerhalb der Kanten (45, 45x) der Deckfläche (41) befinden.

5. Pulswellendetektor nach einem der Ansprüche 1 bis 4, ferner umfassend:
substratseitige Anschlussabschnitte (53) zum elektrischen Verbinden mit Anschlussabschnitten (27), die an Enden in der einen Richtung der Sensorchips (22) vorgesehen sind, wobei die substratseitigen Anschlussabschnitte (53) auf dem Substrat (50) vorgesehen sind; und
leitende Elemente (28) zum Verbinden der Anschlussabschnitte (27) der Sensorchips (22) und der substratseitigen Anschlussabschnitte (53), wobei die leitenden Elemente (28) an Positionen angeordnet sind, die mit den Kanten (45, 45x) der Deckfläche (41) in einer Draufsicht in vertikaler Richtung gesehen nicht überlappen.

6. Messvorrichtung (100) für biologische Informationen, umfassend:
den Pulswellendetektor nach einem der Ansprüche 1 bis 5; und
einen Berechnungsabschnitt für biologische Informationen zum Berechnen von biologischen Informationen basierend auf Druckpulswellen, die von dem Druckpulswellensensor (10, 12) erfasst werden.

## Revendications

1. Détecteur d'ondes pulsatiles comprenant :
un capteur d'ondes pulsatiles de pression (10, 12) comportant :
des puces de capteur (22) ayant chacune une rangée d'éléments (25) comportant une pluralité d'éléments de détection de pression (24) agencés dans une direction ;
un substrat (50) sur lequel les puces de capteur (22) sont respectivement fixées ;
une pièce de protection (40) pour protéger le substrat (50) et les puces de capteur (22) ;
un coussin gonflable (70) ;
une section d'entraînement en rotation (60) fixée au coussin gonflable (70) et supportant de manière rotative le capteur d'ondes pulsatiles de pression (10, 12) ; et
un matériau de remplissage (55) rempli dans un espace entre des faces de détection (26) des puces de capteur (22) sur lesquelles les éléments de détection de pression (24) sont formés et des sections d'ouverture (42) prévues dans la pièce de protection (40), les sections d'ouverture (42) étant disposées sur un côté opposé à un côté des puces de capteur (22) sur lequel le substrat (50) est fixé et étant disposées à des positions opposées aux faces de détection (26) dans une direction verticale perpendiculaire aux faces de détection (26),
dans lequel la section d'entraînement en rotation (60) comporte un mécanisme de rotation (61) pour faire tourner le capteur d'ondes pulsatiles de pression (10, 12) autour d'une direction orthogonale à ladite direction et à la direction verticale, dans lequel
une face périphérique extérieure de la pièce de protection (40) comporte :
une face supérieure (41) dans laquelle les sections d'ouverture (42) sont formées ; et
des faces inclinées (44) qui se poursuivent jusqu'aux deux bords (45x) dans ladite direction de la face supérieure (41) et sont inclinées par rapport à des faces d'ouverture des sections d'ouverture (42), des angles d'inclinaison (θ) des faces inclinées (44) par rapport aux faces d'ouverture étant réglés à une valeur supérieure à une valeur maximale d'un angle de rotation du capteur d'ondes pulsatiles de pression (10, 12) que le mécanisme de rotation (61) peut faire tourner.

2. Détecteur d'ondes pulsatiles selon la revendication 1, dans lequel
la pièce de protection (40) est constituée d'un matériau dont la dureté est supérieure à la dureté du matériau de remplissage (55).

3. Détecteur d'ondes pulsatiles selon la revendication 2, dans lequel
la pièce de protection (40) est en céramique.

4. Détecteur d'ondes pulsatiles selon l'une quelconque des revendications 1 à 3, dans lequel
la face périphérique extérieure de la pièce de protection (40) comprend en outre des faces verticales (46) perpendiculaires aux faces d'ouverture des sections d'ouverture (42), et
les faces verticales (46) sont situées sur un côté des puces de capteur (22) plutôt que sur un côté des faces d'ouverture dans la direction verticale, et se chevauchent avec des bords de la face supérieure de la pièce de protection (40) ou situées à l'extérieur des bords (45, 45x) de la face supérieure (41) dans une vue en plan dans la direction verticale.

5. Détecteur d'ondes pulsatiles selon l'une quelconque des revendications 1 à 4, comprenant en outre :
des sections de borne côté substrat (53) pour la connexion électrique à des sections de borne (27) prévues aux extrémités dans ladite direction des puces de capteur (22), les sections de borne côté substrat (53) étant prévues sur le substrat (50) ; et
des éléments conducteurs (28) pour connecter les sections de borne (27) des puces de capteur (22) et les sections de borne côté substrat (53), les éléments conducteurs (28) étant disposés à des positions qui ne se chevauchent pas avec les bords (45, 45x) de la face supérieure (41) dans une vue en plan dans la direction verticale.

6. Dispositif de mesure d'informations biologiques (100) comprenant :
le détecteur d'ondes pulsatiles selon l'une quelconque des revendications 1 à 5 ; et
une section de calcul d'informations biologiques pour calculer des informations biologiques sur la base des ondes pulsatiles de pression détectées par le capteur d'ondes pulsatiles de pression (10, 12).
